# EUROPÄISCHE PATENTSCHRIFT

(11) **EP 0 957 082 B1**
(45) Veröffentlichungstag und Bekanntmachung des Hinweises auf die Patenterteilung: **10.08.2005**
(21) Anmeldenummer: 99108315.5
(22) Anmeldetag: 28.04.1999
(51) Int. Cl.: C07C 231/02, C07C 233/18

(54) **Verfahren zur kontinuierlichen Herstellung von Hydroxyalkylamiden**
Process for the continuous preparation of hydroxyalkylamides
Procédé pour la préparation continue d'hydroxyalcoylamides

(30) Priorität: 15.05.1998 DE 19821883
(43) Veröffentlichungstag der Anmeldung: 17.11.1999
(73) Patentinhaber: Degussa AG, 40474 Düsseldorf (DE)
(72) Erfinder: Elm, Rainer Dr., 45772 Marl (DE); Weihrauch, Thomas Dr., 48249 Dülmen (DE); Behrendt, Klaus Dr., 44628 Herne (DE); Herda, Silvia, 44623 Herne (DE)

(56) Entgegenhaltungen:
- EP-A- 0 068 281
- EP-A- 0 322 834
- EP-A- 0 473 380
- DE-A- 2 509 273

## Beschreibung

Die Erfindung betrifft ein Verfahren zur kontinuierlichen Herstellung von Hydroxylamiden auf der Basis von Carbonsäureestern und Hydroxylaminen.

In der DE-OS 25 09 237 wird ein Verfahren zur Herstellung von Hydroxyalkylamiden beschrieben. Es werden dabei Ester und Hydroxyalkylamide im Lösungsmittel, sowie lösungsmittelfrei diskontinuierlich zum Endprodukt umgesetzt. Die Reaktion dauert mehrere Stunden.

In der EP-A-0 473 380 wird das Verfahren verbessert. Die Temperatur wird so gewählt, daß während der Reaktion ein Kristallbrei entsteht. Die teilweise Kristallisation führt zu Ausbeuteverbesserungen, da die störende Bildung von Dimeren des Zielmoleküls, die mit der Abspaltung von Hydroxyalkylamin einhergeht, zurückgedrängt wird. Auch hier wird diskontinuierlich gearbeitet. Die Reaktionszeit liegt im Bereich mehrer Stunden.

Diese bisher praktizierten Synthesemethoden haben verschiedene Nachteile: Wenn im Lösemittel gearbeitet wird, muß das Lösemittel nachträglich wieder entfernt werden.

Die übliche Herstellung macht ein langsames Zutropfen des Hydroxyalkylamins erforderlich.

Die Technik des Arbeitens im Kristallbrei erfordert eine genaue Korrelation von Viskosität und Temperatur, da auch eine zu starke Kristallbildung die Ausbeute reduziert. Zudem ist die Austragung des Kristallbreis aus dem Reaktor und die sich anschließende Compoundierung des Materials aufwendig.

Aufgabe der vorliegenden Erfindung ist es daher, ein weniger aufwendiges, einfaches, kontinuierlich arbeitendes Verfahren zur Herstellung von Hydroxyalkylamiden zur Verfügung zu stellen, das die genannten Nachteile nicht aufweist.

Überraschenderweise wurde gefunden, daß die Herstellung von Hydroxyalkylamiden kontinuierlich in einem Extruder oder Intensivmischer durchgeführt werden kann.

Gegenstand der Erfindung ist ein Verfahren zur kontinuierlichen Herstellung von Hydroxyalkylamiden aus Carbonsäureestern und Alkanolaminen, wobei die Reaktion der Einsatzstoffe im Extruder oder Intensivmischer durch intensive Durchmischung und kurzzeitige Reaktion unter einer Minute bei Wärmezufuhr, wobei die Temperatur zu Beginn der Reaktion mindestens 100 °C bis 250 °C beträgt und anschließend abnimmt, und gleichzeitiger Abführung des entstehenden Alkohol erfolgt und danach das Endprodukt isoliert wird.

Das erfindungsgemäße Verfahren ist prinzipiell auf keine spezifischen Carbonsäureester und Alkanolamiden als Einsatzstoffe zur kontinuierlichen Herstellung von Hydroxyalkylamiden beschränkt.

Das Prinzip des Verfahrens besteht darin, daß die Einsatzstoffe kontinuierlich z. B. in einem Intensivkneter, einem Ein- oder Mehrschneckenextruder, Planetwalzenextruder, insbesondere in einem Zweischneckenextruder, kurzzeitig auf hohe Temperatur erhitzt werden, wobei durch Vakuum oder über Ausstrippen mit Gas der entsprechende Alkohol entfernt und anschließend das Herstellungsprodukt isoliert wird. Die Isolierung wird bevorzugt durch anschließende schnelle Abkühlung durchgeführt.

Überraschend war es, daß die Umsetzung, die im diskontinuierlichen Verfahren mehrere Stunden benötigt, in kurzer Zeit im hohen Maße abläuft. Der nach dem Stand der Technik schwierig zu beherrschende Schritt der Bildung eines Kristallbreis gezielter Viskosität, ist hier nicht notwendig. Die Bildung von Dimeren kann bei Einstellung der entsprechenden Parameter nahezu vollständig unterdrückt werden, obwohl zu Beginn des Verfahrens hohe Temperaturen angewandt werden, die die Dimerenbildung forcieren sollten.

Je nach Syntheseziel können jedoch auch Verfahrensprodukte hergestellt werden, die neben Monomeren auch zusätzlich Dimere enthalten. Es besteht auch die Möglichkeit Dimere als Hauptprodukt herzustellen.

Von prinzipieller Natur ist die Tatsache, daß kurzzeitige thermische Belastung im Extruder oder Intensivmischer zu Beginn der Reaktion ausreicht, um die Reaktionspartner oder weitestgehendst umzusetzen.

Im Allgemeinen ist eine Temperatur zu Beginn von mindestens 100 °C, bevorzugt 150 °C, erforderlich.

In einer bevorzugten Ausführungsform des Verfahrens erfolgt die Reaktion in einem Extruder oder Intensivmischer mit mehreren gleichen oder verschiedenen Gehäusen, die unabhängig voneinander thermisch gesteuert werden können. Dies wird ermöglicht durch geeignete Bestückung der Mischkammern bzw. Zusammensetzung der Schneckengeometrie sowie durch intensive rasche Durchmischung bei gleichzeitigem intensiven Wärmeaustausch. Dadurch wird eine gleichmäßige Durchströmung in Längsrichtung mit möglichst einheitlicher Verweilzeit gewährleistet. Der Wärmeaustausch erfolgt durch eine unterschiedliche Temperierung in den einzelnen Gerätegehäusen oder -abschnitten. Die Temperatur in den einzelnen Gehäusen variiert zu Beginn der Reaktion von 100 °C - 250 °C und nimmt anschließend ab.

Die Einsatzstoffe werden in der Regel in getrennten Produktströmen zudosiert, können jedoch auch teilweise gebündelt zugeführt werden. Es ist aber auch möglich nur einen Eduktstrom zu dosieren, der alle Edukte enthält. Hydroxylamine bzw. Mischungen verschiedener Hydroxylamine, und/oder Carbonsäureester und/oder Katalysatoren, und/oder Zuschlagstoffe wie Verlaufsmittel und Stabilisatoren können zu einem Produktstrom zusammengefaßt werden; ebenso Carbonsäureester, Mischungen von Estern sowie die oben genannte Zuschlagstoffe und Katalysatoren.

Die Stoffströme können auch geteilt werden und so in unterschiedlichen Anteilen an verschiedenen Stellen dem Extruder oder Intensivmischer zugeführt werden. Auf diese Weise werden gezielt Konzentrationsgradienten eingestellt, was die Vollständigkeit der Reaktion herbeiführen kann.

Die Eintrittsstelle der Produktströme in der Reihenfolge kann variabel und zeitlich versetzt gehandhabt werden.

Die Vollständigkeit der Reaktion wird durch eine Abführung der bei der Amidierung entstehenden Alkohole gewährleistet. Diese Abführung erfolgt bevorzugt durch das Abziehen der Alkohole mittels Vakuum über Öffnungen in den Gehäusen des Extruders oder Intensivmischers und/oder durch Überleiten eines Gasstroms über das intensiv gemischte Reaktionsgemisch, wobei die flüchtigeren Alkohole vom Gasstrom ausgetragen werden.

Die Reaktion kann durch Katalysatoren beschleunigt werden. Geeignet sind Hydroxyde und/oder Alkoholate von Alkalimetallen wie z. B. Natrium- oder Kaliumhydroxid, Natrium- oder Kaliumethanolat, quaternäre Ammoniumhydroxide, Alkoxyde und/oder andere starke Basen. Die Konzentration beträgt 0,01 bis %, bevorzugt 0,1 bis 1 % bezogen auf den eingesetzten Carbonsäureester.

Die Anordnung von Vakuum-Domen bzw. Gas-Überleit-Stellen kann variabel erfolgen und richtet sich nach Art der Edukte und der entstehenden Alkohole. Auch eine zusätzliche, dem eigentlichen Reaktionsteil nachgeschaltete Stelle zur Entfernung von Alkohol-Restmengen ist möglich.

Die der schnellen Reaktion nachgeschaltete, bevorzugt schnelle, intensive Abkühlung kann in dem Reaktionsteil integriert sein, in Form einer mehrgehäusigen Ausführungsform wie bei Extrudern oder Conterna-Maschinen. Eingesetzt werden können außerdem: Rohrbündel, Rohrschlangen, Kühlwalzen, Luftförderer und Transportbänder aus Metall.

Die Konfektionierung wird je nach Viskosität des Endproduktes zunächst durch weitere Abkühlung mittels entsprechender vorgenannter Gerätschaften auf eine geeignete Temperatur gebracht. Dann erfolgt die Pastillierung oder aber eine Zerkleinerung in eine gewünschte Partikelgröße mittels Walzenbrecher, Stiftmühle, Hammermühle, Schuppwalzen oder ähnlichem.

Eine bevorzugte Ausführungsform der Erfindung ist ein Verfahren zur kontinuierlichen Herstellung von Hydroxyalkylamiden der Formel I aus Carbonsäureestern und Alkanolaminen 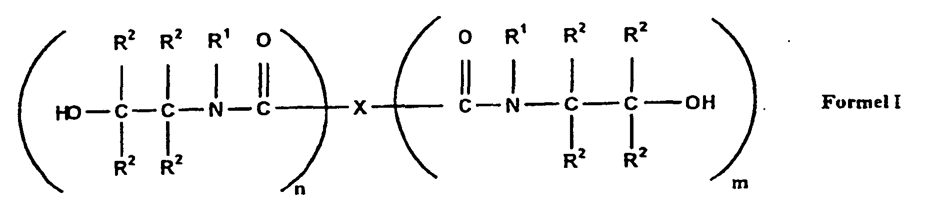 wobei die Substituenten die folgenden Bedeutungen aufweisen:
X: Bindung, Wasserstoff für m = 0,
Alkylen-, Aryl-, Alkenyl-, AJkoxycarbonyl- oder Carboxylalkenylrest mit 1-24 Kohlenstoffatomen;
- R¹:: Wasserstoff, Alkyl-, Alkenyl, Aryl- oder Aralkylrest mit 1-24 Kohlenstoffatomen, diese heteroatomsubstituierten Reste oder

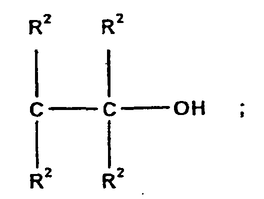

- R²:: unabhängig voneinander gleiche oder verschiedene Reste, ausgewählt aus Wasserstoff, Alkyl-, Aryl-, Aralkyl- oder Alkenylrest mit 1-24 Kohlenstoffatomen oder diese heteroatomsubstituierten Reste;
- n:: ganze Zahl von 1-10;
- m:: ganze Zahl von 0-2.

Besonders geeignet ist das Verfahren zur Herstellung von

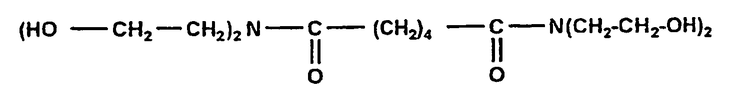

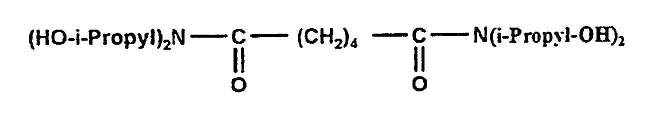

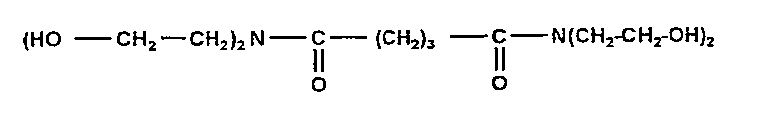

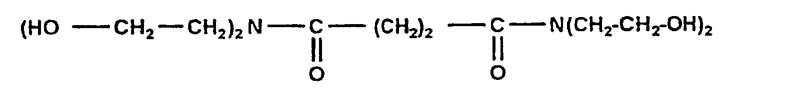

### Allgemeine Herstellvorschrift

In die Einzugsgehäuse eines Doppelschneckenextruders wird der Carbonsäureester bei einer Temperatur von 25 bis 220 °C eingespeist, wobei gleichzeitig das Hydroxyalkylamin mit einer Temperatur von 25 bis 200 °C zudosiert wird. Einer der beiden Stoffströme enthält den gelösten Katalysator. Einer von beiden Stoffströmen kann bei Bedarf (zu geringer Umsatz) aufgesplittet werden. Die Zudosierung dieses Stromes erfolgt dann in der Weise, daß die Hauptmenge (über 50 %) in die Einzugsgehäuse dosiert wird, während der kleinere Strom in eines der folgenden Gehäuse eingespeist wurde, um so eine Nachreaktion zu ermöglichen.

Der eingesetzte Extruder setzt sich aus verschiedenen Gehäusen zusammen, wobei üblicherweise mindestens die Hälfte thermisch gesteuert werden kann.

Bei einem Extruder mit 8 Gehäusen werden die Temperaturen wie folgt eingestellt:
G1: 100 - 220 °C, G2 bis G6: 80 - 220 °C, G7, G8: 60- 160 °C.

Wird ein Eduktstrom geteilt, wird die Temperatur der zweiten Einspeisstellt genauso oder ähnlich wie in G1 gewählt.

Oberhalb von zwei Gehäusen nach G1 befinden sich Vakuum-Dome zum Abziehen des während der Reaktion entstehenden Alkohols.

Alle Temperaturen stellen Soll-Temperaturen dar; die Temperatureinstellung in den Knetergehäusen erfolgt durch elektrische Heizung und Wasser- oder Luftkühlung (pneumatisch).

Die Drehzahl der Doppelschnecke, die auf der Gesamtlänge aus Förder- und Knetelementen aufgebaut ist, beträgt 10 bis 380 Upm.

Das Verhältnis der Ester-Gruppen zu Hydroxylamin-Molekülen beträgt 1 zu 0,5 bis 1,5, bevorzugt 1 zu 1.

Das Reaktionsprodukt wird entweder abgekühlt, anschließend zerkleinert oder formiert und abgefüllt.

### Beispiel:

Amidester (ADE) aus der Umsetzung von Adipinsäuredimethylester (ADM) mit Diethanolamin (DEA).
Es werden zwei Mol DEA pro Mol ADM umgesetzt. Als Katalysator fungiert KOH (0,2 % bezogen auf die ADM-Menge).
Stoffstrom 1 besteht aus ADM, Stoffstrom 2 aus DEA, in dem das KOH gelöst ist. Die Stoffströme werden nicht geteilt. Die Zugabe erfolgt im ersten Gehäuse eines Zweischneckenextruders. An zwei Vakuum-Domen wird das entstehende Methanol weitgehend abgesaugt. Das entstehende Produkt wird auf ein Kühlband aufgebracht. Die Verweilzeit im Extruder beträgt unter einer Minute.

Zusammensetzung des Produktes laut ¹³C-NMR:

| | |
|---|---|
| DEA | 2,0 % |
| ADE | 97,5 % |
| CH₃OH | 0,5 % |

## Patentansprüche

1. Verfahren zur kontinuierlichen Herstellung von Hydroxyalkylamiden aus Carbonsäureestern und Alkanolaminen, wobei die Reaktion der Einsatzstoffe im Extruder oder Intensivmischer durch intensive Durchmischung und kurzzeitige Reaktion unter einer Minute bei Wärmezufuhr, wobei die Temperatur zu Beginn der Reaktion mindestens 100 °C bis 250 °C beträgt und anschließend abnimmt, und gleichzeitiger Abführung des entstehenden Alkohol erfolgt und danach das Endprodukt isoliert wird.

2. Verfahren nach Anspruch 1,
**dadurch gekennzeichnet,**
**dass** die Reaktion ohne Lösungsmittel erfolgt.

3. Verfahren nach den Ansprüchen 1-2,
**dadurch gekennzeichnet,**
**dass** die Reaktion im Einwellen-, Mehrwellen- oder Planetwalzenextruder erfolgt.

4. Verfahren nach Anspruch 3,
**dadurch gekennzeichnet,**
**dass** die Reaktion in einen Zweischneckenextruder erfolgt.

5. Verfahren nach den Anprüchen 1-2,
**dadurch gekennzeichnet,**
**dass** die Reaktion im Intensivkneter oder statischen Mischer erfolgt.

6. Verfahren nach den Ansprüchen 1-5,
**dadurch gekennzeichnet,**
**dass** die Reaktion in Gegenwart von Katalysatoren und/oder Zuschlagsstoffen erfolgt.

7. Verfahren nach den Ansprüchen 1-6,
**dadurch gekennzeichnet,**
**dass** die Reaktion in einem Extruder oder Intensivmischer mit mehreren gleichen oder verschiedenen Gehäusen, die unabhängig voneinander thermisch gesteuert werden können, erfolgt.

8. Verfahren nach den Ansprüchen 1 bis 7,
**dadurch gekennzeichnet,**
**dass** die Einsatzstoffe und/oder Zusatzstoffe einzeln oder gebündelt eingesetzt werden.

9. Verfahren nach den Ansprüchen 1 bis 8,
**dadurch gekennzeichnet,**
**dass** der entstehende Alkohol durch Vakuum und/oder durch Überleiten eines inerten Gasstromes über das intensiv gemischte Reaktionsgemisch abgeführt wird.

10. Verfahren nach den Ansprüchen 1 bis 9,
**dadurch gekennzeichnet,**
**dass** die Endprodukte durch schnelle intensive Abkühlung erhalten werden.

11. Verfahren nach mindestens einem der Ansprüche 1 bis 10 zur kontinuierlichen Herstellung von Hydroxyalkylamiden der Formel I aus Carbonsäureestern und Alkanolaminen 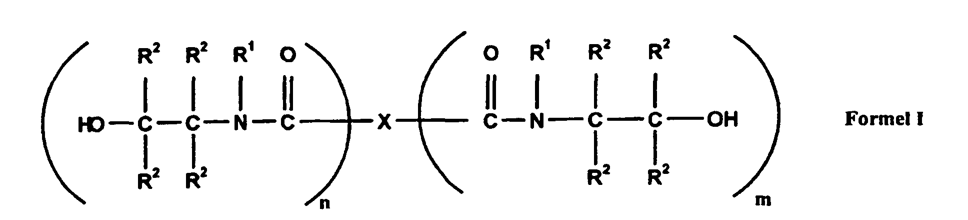 wobei die Substituenten die folgenden Bedeutungen aufweisen:
X: Bindung, Wasserstoff für m = 0,
Alkylen-, Aryl-, Alkenyl- ,Alkoxycarbonyl- oder Carboxyalkenylrest mit 1 bis 24 Kohlenstoffatomen;
R¹: Wasserstoff, Alkyl-, Alkenyl-, Aryl- oder Aralkylrest mit 1 bis 24 Kohlenstoffatomen, diese heteroatomsubstituierten Reste oder 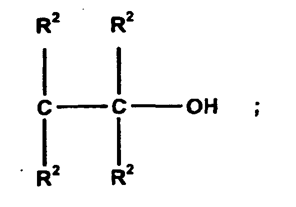
R²: unabhängig voneinander gleiche oder verschiedene Reste, ausgewählt aus Wasserstoff, Alkyl-, Aryl-, Aralkyl- oder Alkenylrest mit 1 bis 24 Kohlenstoffatomen oder diese heteroatomsubstituierten Reste;
n: ganze Zahl von 1 bis 10;
m: ganze Zahl von 0 bis 2.

12. Verfahren nach mindestens einem der Ansprüche 1 bis 11 zur Herstellung von

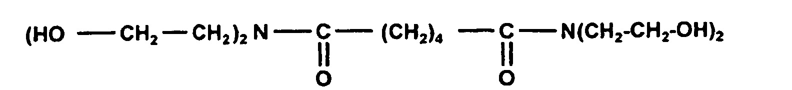

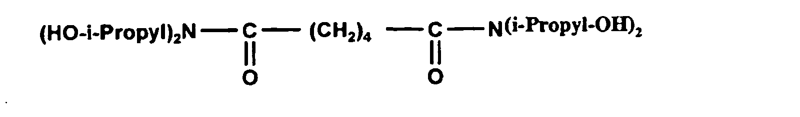

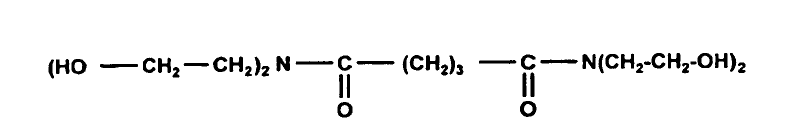

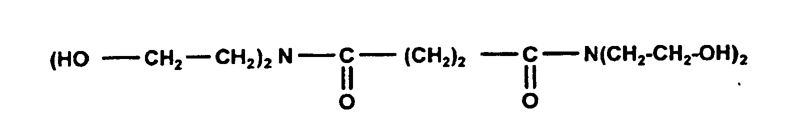

## Claims

1. A process for the continuous preparation of a hydroxyalkylamide from a carboxylic ester and an alkanolamine, wherein the reaction of the starting materials is carried out in an extruder or intensive mixer by intensive mixing and brief reaction for less than one minute with supply of heat, the temperature at the beginning of the reaction being from at least 100° to 250°C and decreasing from there on, and simultaneous removal of the alcohol formed takes place and the final product is then isolated.

2. A process according to claim 1, **characterized in that** the reaction is carried out without solvent.

3. A process according to either of claims 1 and 2, **characterized in that** the reaction is carried out in a single-screw, multiscrew or planetary-gear extruder.

4. A process according to claim 3, **characterized in that** the reaction is carried out in a twin-screw extruder.

5. A process according to either of claims 1 and 2, **characterized in that** the reaction is carried out in an intensive kneader or static mixer.

6. A process according to any of claims 1 to 5, **characterized in that** the reaction is carried out in the presence of a catalyst and/or an additive.

7. A process according to any of claims 1 to 6, **characterized in that** the reaction is carried out in an extruder or intensive mixer having a plurality of identical or different barrel sections which can be thermally controlled independently of one another.

8. A process according to any of claims 1 to 7, **characterized in that** the starting materials and/or additives are introduced individually or in combination.

9. A process according to any of claims 1 to 8, **characterized in that** the alcohol formed is removed by means of reduced pressure and/or by passing an inert gas stream over the intensively mixed reaction mixture.

10. A process according to any of claims 1 to 9, **characterized in that** the end product is obtained by rapid intensive cooling.

11. A process according to at least one of claims 1 to 10 for the continuous preparation of a hydroxyalkylamide of the formula I from a carboxylic ester and an alkanolamine 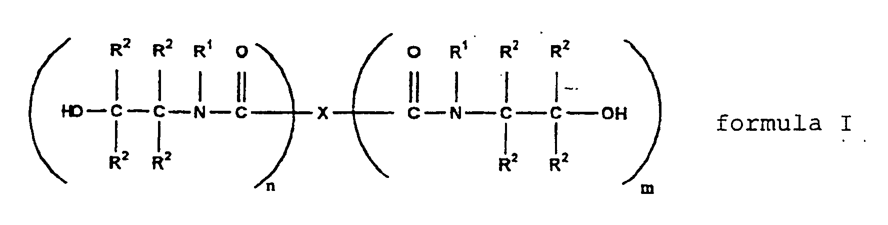 where the substituents have the following meanings:
X: a bond, hydrogen when m = 0,
an alkylene, aryl, alkenyl, alkoxycarbonyl or carboxyalkenyl radical having 1 to 24 carbon atoms;
R¹: hydrogen, an alkyl, alkenyl, aryl or aralkyl radical having 1 to 24 carbon atoms, these heteroatom-substituted radicals or 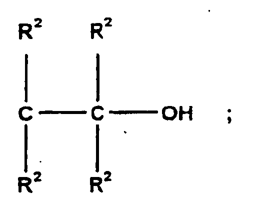
R²: independently of one another, identical or different radicals selected from the group consisting of hydrogen, alkyl, aryl, aralkyl and alkenyl radicals having 1 to 24 carbon atoms and these heteroatom-substituted radicals;
n: integer from 1 to 10;
m: integer from 0 to 2.

12. A process according to at least one of claims 1 to 11 for preparing

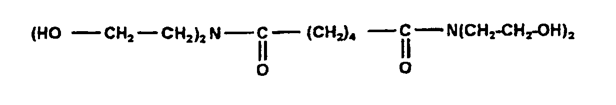

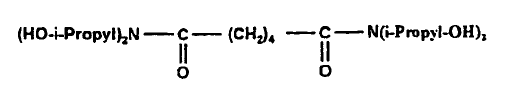

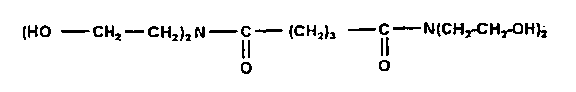

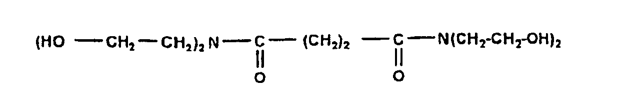

## Revendications

1. Procédé de préparation continue d'hydroxyalkylamides à partir d'esters d'acide carboxylique et d'alcanolamines, selon lequel la réaction entre les matières chargées s'effectue dans une extrudeuse ou dans un mélangeur intensif, au moyen d'un malaxage intensif et d'une réaction de brève durée de moins d'une minute avec un apport de chaleur, la température étant d'au moins 100 °C à 250 °C au début de la réaction et diminuant par la suite) et avec une évacuation simultanée de l'alcool produit, après laquelle le produit final est isolé.

2. Procédé selon la revendication 1,
**caractérisé en ce que**
la réaction s'effectue sans solvant.

3. Procédé selon les revendications 1 et 2,
**caractérisé en ce que**
la réaction s'effectue dans une extrudeuse à un seul arbre, à plusieurs arbres ou à galets planétaires.

4. Procédé selon la revendication 1,
**caractérisé en ce que**
la réaction s'effectue dans une extrudeuse à deux vis.

5. Procédé selon les revendications 1 et 2,
**caractérisé en ce que**
la réaction s'effectue dans un malaxeur intensif ou dans un mélangeur statique.

6. Procédé selon les revendications 1 à 5,
**caractérisé en ce que**
la réaction s'effectue en présence de catalyseurs et/ou de produits d'addition.

7. Procédé selon les revendications 1 à 6,
**caractérisé en ce que**
la réaction s'effectue dans une extrudeuse ou un mélangeur intensif comportant plusieurs boîtiers identiques ou différents qui peuvent être commandés thermiquement indépendamment les uns des autres.

8. Procédé selon les revendications 1 à 7,
**caractérisé en ce que**
les ingrédients et/ou produits d'addition sont mis en oeuvre isolément ou sous forme concentrée.

9. Procédé selon les revendications 1 à 8,
**caractérisé en ce que**
l'alcool produit est évacué au moyen d'un vide et/ou en faisant passer un courant gazeux inerte par-dessus le mélange de réaction malaxé de manière intensive.

10. Procédé selon les revendications 1 à 9,
**caractérisé en ce que**
les produits finals sont obtenus par refroidissement rapide et intensif.

11. Procédé selon l'une au moins des revendications 1 à 10 en vue de la préparation continue d'hydroxyalkylamides selon la formule (I) à partir d'esters d'acide carboxylique et d'alcanolamines 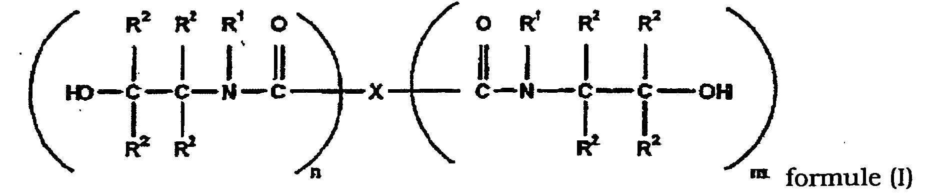 où les substituants ont les significations suivantes :
X : liaison, hydrogène pour m = 0, reste alkylène, aryle, alcényle, alcoxycarbonyle ou carboxyalcényle comportant de 1 à 24 atomes de carbone,
R¹ : hydrogène, reste alkyle, alcényle, aryle ou aralkyle comportant de 1 à 24 atomes de carbone, ces restes substitués par des hétéroatomes ou 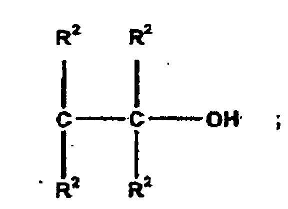
R² : restes identiques ou différents indépendamment les uns des autres, sélectionnés parmi les substances suivantes : hydrogène, reste alkyle, aryle, aralkyle ou alcényle comportant de 1 à 24 atomes de carbone ou ces restes substitués par des hétéroatomes ;
n : nombre entier de 1 à 10 ;
m : nombre entier de 0 à 2.

12. Procédé selon l'une au moins des revendications 1 à 11, pour la préparation de :
